# EUROPEAN PATENT SPECIFICATION

(11) **EP 2 449 887 B1**
(45) Date of publication and mention of the grant of the patent: **06.01.2016**
(21) Application number: 10190118.9
(22) Date of filing: 05.11.2010
(51) Int. Cl.: A23L 1/30, A23K 1/00, A23K 1/18

(54) **Method for preparing pet food containing probiotic micro-organisms**
Verfahren zur Herstellung von Haustierfutter enthaltend probiotische Mikroorganismen
Procédé de préparation d'aliments pour animaux de compagnie contenant des micro-organismes probiotiques

(43) Date of publication of application: 09.05.2012
(73) Proprietor: Nestec S.A., 1800 Vevey (CH)
(72) Inventor: Mercenier, Annick, 1030, BUSSIGNY (CH); Prioult, Guénolée, 1005, LAUSANNE (CH); Nutten, Sophie, 1005, LAUSANNE (CH)
(74) Representative: Rupp, Christian

(56) References cited:
- US-A1- 2005 175 598
- US-A1- 2005 180 962
- US-A1- 2009 274 661
- DATABASE FSTA [Online] INTERNATIONAL FOOD INFORMATION SERVICE (IFIS), FRANkFURT-MAIN, DE; ADAMS C A: "The probiotic paradox: live and dead cells are biological response modifiers.", XP002639353, Database accession no. FS-2010-10-Ad4160 & NUTRITION RESEARCH REVIEWS, vol. 23, no. 1, 2010, page 37,
- DATABASE FSTA [Online] INTERNATIONAL FOOD INFORMATION SERVICE (IFIS), FRANkFURT-MAIN, DE; LIYAN ZHANG ET AL: "Alive and dead Lactobacillus rhamnosus GG decrease tumor necrosis factor-alpha -induced interleukin-8 production in Caco-2 cells.", XP002639354, Database accession no. FS-2005-10-Ad1681 & JOURNAL OF NUTRITION, vol. 135, no. 7, 2005, page 1752,
- DATABASE FSTA [Online] INTERNATIONAL FOOD INFORMATION SERVICE (IFIS), FRANkFURT-MAIN, DE; OUWEHAND A C ET AL: "Adhesion of inactivated probiotic strains to intestinal mucus.", XP002639355, Database accession no. FS-2000-12-Ad1945 & LETTERS IN APPLIED MICROBIOLOGY, vol. 31, no. 1, 2000, pages 82-86,

## Description

The present invention relates to the field of pet food. In particular, the present invention provides method for preparing pet food compositions comprising non-replicating probiotic microorganisms. These non-replicating probiotic micro-organisms are bioactive heat treated probiotic micro-organisms.

The health benefits of probiotics are meanwhile well accepted in the art and were summarized, e.g., by Blum et al. in Curr Issues Intest Microbiol. 2003 Sep;4(2):53-60. Oftentimes probiotics are administered together with prebiotics in symbiotic formulations which may even have enhanced health benefits.

The well-being of domestic animals is closely related to their feeding. Correct feeding should result in a fit and healthy pet. In addition to providing nutritional value, food composition influences the intestinal microflora equilibrium and may lead to or prevent gastrointestinal disorders. Therefore, knowledge on the gastrointestinal tract and digestion processes of healthy animals is integral to the understanding of a practical feeding practice.

Often canine and feline gastrointestinal disorders are linked to bacterial overgrowth and the production of enterotoxins produced by pathogenic bacteria.

During the last few years research has focused on some valuable strains of lactic acid bacteria and their potential use as probiotic agents. Probiotics are considered to be viable microbial preparations which promote mammalian health by preserving the natural microflora in the intestine. Probiotics are thought to attach to the intestinal mucosa, colonize the intestinal tract and thereby prevent attachment of harmful microorganisms thereon. A prerequisite for their action resides in that they have to reach the gut's mucosa in a proper and viable form and especially do not get destroyed by the influence of the low pH prevailing in the stomach. In particular, the physiology of the digestive tract of cats and dogs differs from humans. For example, the average pH in the stomach is 3.4 for dogs and 4.2 for cats.

United States Patent 7189390 describes novel lactic acid bacterial micro-organisms that have been isolated and selected for their probiotic potential and their use for the preparation of pet food compositions intended to improve the health of pets.

US 2005/0180962 describes inactivated probiotic bacteria and methods of use thereof.

Since up to 70% of the immune system is contained within the digestive tract of animals, probiotics not only aid in the digestive health of the animal, but the whole immune system of a pet.

Probiotic bacteria are known to be capable of adhering to intestinal cells and of excluding pathogenic bacteria on intestinal cells. To have this activity, the probiotic bacteria must remain viable in the product until it is consumed. Adding live bacteria into pet food kibble so that they stay viable until the product is consumed and the bacteria arrive viable in the intestinal tract remains to be a challenge and to accomplish this requires significant technical effort.

It would be desirable to have available a pet food composition that is able to deliver probiotic benefits even after longer storage times under critical conditions for the probiotics, while being simple to prepare. It would be preferred if this was achieved by using natural ingredients that are safe to administer without side effects and that are easy to incorporate into pet food compositions using state of the art industrial techniques.

It would also be desirable to further improve the immune boosting effect of probiotics in such preparations.

It would further be desirable to further improve the anti-inflammatory effect of probiotics in such preparations.

The present inventors have addressed this need. It was hence the objective of the present invention to improve the state of the art and to provide a method for preparing pet food compositions that satisfy the needs expressed above.

The present inventors were surprised to see that they could achieve this object by the subject matter of the independent claim. The dependant claims further develop the idea of the present invention.

Accordingly, the present inventors propose a method to provide a pet food composition comprising non-replicating probiotic micro-organisms.

The invention is directed to a method for preparing a pet food composition comprising non-replicating probiotic micro-organisms in an amount corresponding to 10⁶ to 10¹² cfu per serving said method comprising treating the probiotic micro-organisms with a high temperature/ short time (HTST) treatment at a temperature of 120-140°C for 1-30 seconds or a ultra-high temperature (UHT) treatment at a temperature exceeding 135°C for 1-10 seconds, and thus rendering at least 90% of the micro-organisms non-replicating.

In one embodiment of the method said pet food composition is comprising from 4 to 40 weight-% dry weight fat, from 12 to 70 weight-% dry weight carbohydrates, and from 12 to 50 weight-% dry weight proteins.

In one embodiment of the method said pet food composition is comprising from 10 to 20 weight-% dry weight fat, from 30 to 60 weight-% dry weight carbohydrates, and from 20 to 35 weight-% dry weight proteins.

In one embodiment of the method said pet food composition is further comprising from 0.5 to 40 weight-% dry weight, preferably from 0.5 to 30 weight-% dry weight, more preferably from 1 to 20 weight-% dry weight, most preferably from 1 to 10 weight-% dry weight dietary fiber.

In one embodiment of the method the pet food composition is selected from the group consisting of pet foods, nutritional diets for pets, supplements for pets, treats for pets, and food toys for pets such as chewable and consumable toys.

In one embodiment of the method said pet food composition is further comprising prebiotics, for example oligofructose and inulin.

In one embodiment of the method said pet food composition at least 95 %, preferably at least 98 %, more preferably at least 99 %, most at least 99.9 %, ideally all of the probiotics are non-replicating.

In one embodiment of the method the probiotic micro-organisms are selected from the group consisting of bifidobacteria, lactobacilli , propionibacteria, or combinations thereof, for example Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus reuteri, Lactobacillus rhamnosus, Lactobacillus johnsonii Lactobacillus plantarum, Lactobacillus fermentum, Lactococcus lactis, Streptococcus thermophilics, Lactococcus lactis, Lactococcus diacetylactis, Lactococcus cremoris, Lactobacillus bulgaricus, Lactobacillus helveticusr Lactobacillus delbrueckii , Escherichia coli and/or mixtures thereof.

In one embodiment of the method the probiotic micro-organisms are selected from the group consisting of Bifidobacterium longum NCC 3001 (ATCC BAA-999), Bifidobacterium longum NCC 2705 (CNCM I-2618), Bifidobacterium breve NCC 2950 (CNCM I-3865), Bifidobacterium lactis NCC 2818 (CNCM I-3446), Lactobacillus johnsonii La 1 (CNCM I-1225), Lactobacillus paracasei NCC 2461 (CNCM I-2116), Lactobacillus rhamnosus NCC 4007 (CGMCC 1.3274), Lactobacillus reuteri DSM17983, Lactobacillus reuteri ATCC 55730, Streptococcus thermophilus NCC 2059 (CNCM I-4153), Lactobacillus casei NCC 1825 (ACA-DC 6002), Escherichia coli Nissle (DSM 6601), Lactobacillus bulgaricus NCC 15 (CNCM I-1198), Lactococcus lactis NCC 2287 (CNCM I-4154), or combinations thereof.

In one embodiment of the method said pet food composition is containing 0,005 mg - 1000 mg non- replicating micro-organisms per daily dose.

Pet food compositions comprise a variety of compositions e.g., foods, nutritional diets, supplements, treats, and food toys such as chewable and consumable toys.

Pets include domestic animals such as dogs, cats, birds, rabbits, guinea pigs, goats, cows, horses, pigs, for example.

The compositions can be foods having any suitable form, e.g., liquid or solid foods. When the foods are liquid foods, the non-replicating probiotic micro-organisms may be admixed with the foods. When the foods are solid foods, the non-replicating probiotic micro-organisms may be coated on the foods, incorporated into the foods, or both. When coated onto or incorporated into the foods, the non-replicating probiotic micro-organisms can be homogeneously or non-homogeneously dispersed into or onto the foods.

The pet food compositions typically contain a carbohydrate fraction, a protein fraction and a fat fraction.

The percentages are - if not otherwise indicated - weight-% on a dry matter basis.

The pet food composition may comprise from 12% to 70%, preferably from 16% to 65%, more preferably from 20% to 60%, most preferably from 30% to 60% of a carbohydrate fraction; from 12% to 50%, preferably from 16% to 45%, more preferably from 18% to 40%, most preferably from 20% to 35% of a protein fraction; and from 4% to 40%, preferably from 6% to 30%, more preferably from 8% to 25%, most preferably from 10% to 20% of a fat fraction.

For some pet food compositions, e.g., pet treats, the compositions may contain from 1 to 12% fat, typically in the form of a coating to enhance palatability.

The pet food composition may also comprise dietary fiber from 0.5% to 40%, preferably from 0.5% to 30%, more preferably from 1% to 20%, most preferably from 1% to 10%

Nutritional balancing agents (i.e., vitamins, minerals, trace elements, and combinations thereof) may be added. Typically such nutritional balancing agents may be added in an amount from 0.01% to 15%, preferably from 0.05% to 10%, more preferably from 1% to 5%, most preferably from 1% to 3%

Specific suitable amounts for each ingredient in a composition will depend on a variety of factors such as the species of animal consuming the composition; the particular ingredients included in the composition; the age, weight, general health, sex, and diet of the animal; the animal's consumption rate; and the like. Thus, the ingredient amounts may vary widely, and may even deviate from the proportions given herein. Selection of such components and amounts of the components are within the scope of the skilled artisan. For some companion animals such as dogs and cats, the American Feed Control Officials (AAFCO) provides recommended amounts of such ingredients.

The protein food source may be obtained from a variety of sources such as plants, animals, or both. Animal protein includes meat, meat by products, dairy, and eggs. Meats include the flesh from poultry, fish, and animals such as cattle, swine, sheep, goats, and the like. Meat by products include lungs, kidneys, brain, livers, stomachs, and intestines. The protein food ingredient may also be free amino acids and/or peptides. Preferably, the protein food ingredient comprises meat, a meat by-product, dairy products, or eggs.

The fat and carbohydrate food source may be obtained from a variety of sources such as animal fat, fish oil, vegetable oil, meat, meat by products, grains, other animal or plant sources, and mixtures thereof. Grains include wheat, corn, barley, and rice.

The fiber food ingredient may be obtained from a variety of sources such as vegetable fiber sources, e.g., cellulose, beet pulp, peanut hulls, and soy fiber.

Particularly when the composition is an animal food, vitamins and minerals preferably are included in amounts required to avoid deficiency and maintain health. These amounts are readily available in the art. The National Research Council (NRC) provides recommended amounts of such ingredients for farm animals. See, e.g., Nutrient Requirements of Swine (10th Rev. Ed., Nat'l Academy Press, Wash. D.C., 1998), Nutrient Requirements of Poultry (9th Rev. Ed., Nat'l Academy Press, Wash. D.C., 1994), Nutrient Requirements of Horses (5th Rev. Ed., Nat'l Academy Press, Wash. D.C., 1989), etc. The American Feed Control Officials (AAFCO) provides recommended amounts of such ingredients for dogs and cats. See American Feed Control Officials, Inc., Official publication, pages 126 140 (2003). Vitamins generally useful as food additives include vitamin A, vitamin B1, vitamin B2, vitamin B6, vitamin B12, vitamin C, vitamin D, vitamin E, vitamin H (biotin), vitamin K, folic acid, inositol, niacin, and pantothenic acid. Minerals and trace elements generally useful as food additives include calcium, phosphorus, sodium, potassium, magnesium, copper, zinc, choline, and iron.

The compositions may contain additional ingredients such as vitamins, minerals, fillers, palatability enhancers, binding agents, flavors, stabilizers, emulsifiers, sweeteners, colorants, buffers, salts, coatings, and the like known to skilled artisans. Stabilizers include substances that tend to increase the shelf life of the composition such as preservatives, synergists and sequestrants, packaging gases, stabilizers, emulsifiers, thickeners, gelling agents, and humectants. Examples of emulsifiers and/or thickening agents include gelatin, cellulose ethers, starch, starch esters, starch ethers, and modified starches. Specific amounts for each composition component, food ingredient, and other ingredients will depend on a variety of factors such as the particular components and ingredients included in the composition; the species of patient; the patient's age, body weight, general health, sex, and diet; the patient's consumption rate; the type of disease being treated (if any); and the like. Therefore, the ingredient amounts may vary widely and may deviate from the preferred proportions described herein. The amount of such additives in a composition typically is up to 5% by weight.

The compositions may be or may contain additional ingredients intended to maintain or improve the health of the animal, e.g., supplements, medications, herbs, holistic drugs and compositions, and the like.

Supplements include a feed used with another feed to improve the nutritive balance or performance of the total. Supplements include compositions that are fed undiluted as a supplement to other feeds, offered free choice with other parts of an animal's ration that are separately available, or diluted and mixed with an animal's regular feed to produce a complete feed. The AAFCO provides a discussion relating to supplements in the American Feed Control Officials, Inc. Official Publication, page 220 (2003). Supplements may be in various forms including powders, liquids, syrups, pills, encapsulated compositions, and the like.

Treats include compositions that are given to an animal to entice the animal to eat during a non meal time, e.g., dog bones for canines. Treats may be nutritional wherein the composition comprises one or more nutrients, and may have a composition as described above for food. Non nutritional treats encompass any other treats that are non toxic. The non-replicating probiotic micro-organisms are coated onto the treat, incorporated into the treat, or both.

Toys include chewable toys such as artificial bones. The non-replicating probiotic micro-organisms can form a coating on the surface of the toy or on the surface of a component of the toy, be incorporated partially or fully throughout the toy, or both. The non-replicating probiotic micro-organisms are orally accessible by the intended user. There are a wide range of suitable toys currently marketed, e.g., U.S. Pat. No. 5,339,771, U.S. Pat. No. 5,419,283, and references disclosed therein. Disclosed are both partially consumable toys, e.g., toys comprising plastic components, and fully consumable toys, e.g., rawhides and various artificial bones. Further, toys are disclosed for both human and non-human use, particularly for companion, farm, and zoo animal use, and particularly for dog, cat, or bird use.

In preparing the compositions, the components are adjusted so that the non-replicating probiotic micro-organisms are present in the composition at a concentration of at least 0.01%, preferably from 0.01% to 4%, most preferably from 0.5% to 2% by weight of the composition. The non-replicating probiotic micro-organisms may be incorporated into the composition during the processing of the formulation, such as during and/or after mixing of other components of the composition. Distribution of these components into the composition is accomplished by conventional means.

Compositions (particularly foods) can be prepared in a dry form using conventional processes. Dry ingredients, including animal protein sources, plant protein sources, grains, etc. can be ground and mixed together. Moist or liquid ingredients, including fats, oils, animal protein sources, water, etc. are then added to and mixed with the dry mix. The mixture is then processed into kibbles or similar dry pieces. Kibble is often formed using an extrusion process in which the mixture of dry and wet ingredients is subjected to mechanical work at a high pressure and temperature, and forced through small openings and cut off into kibble by a rotating knife. The wet kibble is then dried and optionally coated with one or more topical coatings which may include flavors, fats, oils, powders, and the like. Kibble also can be made from the dough using a baking process, rather than extrusion, wherein the dough is placed into a mold before dry-heat processing.

The non-replicating probiotic micro-organisms may be added to the pet food composition in its normal preparation procedure such as mixing, extrusion, baking and the like or is preferably added after its preparation post extrusion, such as by spraying or coating the surface of the food. This is particularly desirable for dry foods wherein the extruded strands are contacted with the non-replicating probiotic micro-organisms (or a solution comprising the non-replicating probiotic micro-organisms) by spraying or coating the extruded strands before the strands are cut into a kibble, or the kibble is contacted with the non-replicating probiotic micro-organisms (or a solution comprising the non-replicating probiotic micro-organisms) by spraying, coating or dipping the kibble per se.

For topical application to a food, the non-replicating probiotic micro-organisms are mixed with a carrier composition to facilitate application to the surface of the food composition. For example, a liquid, slurry, light gel, or watery solid can all be utilized as a carrier for the compound(s) of this composition. A standard spraying or dipping apparatus is employed to apply the compound(s) to the surface of the food composition. An example of such a carrier is a minced animal by-product treated with proteases in conjunction with amino acids, reducing sugar(s) and thiamin. The carrier is then mixed with the non-replicating probiotic micro-organisms and coated onto a kibble, thereby preparing a very palatable and acceptable dry food. The non-replicating probiotic micro-organisms may simply be mixed with a commercial liquid palatant enhancer or other flavor composition to create a novel flavor palatant which can then be topically applied to the composition. Suitable commercial liquid palatant enhancers for use with the non-replicating probiotic micro-organisms include any known or commercially available liquid palatant enhancers commercially available from pet food palatant enhancer or other flavor suppliers known to those of skill in the art.

Compositions (particularly foods) can be prepared in a canned or wet form using conventional pet food processes. Ground animal (e.g., mammal, poultry, fish and/or seafood) proteinaceous tissues can be mixed with the other ingredients, including fish oils, cereal grains, other nutritionally balancing ingredients, special purpose additives (e.g., vitamin and mineral mixtures, inorganic salts, cellulose and beet pulp, bulking agents, and the like). Water sufficient for processing may also be added. The wet form ingredients are typically mixed in a vessel suitable for heating while blending the components. Heating of the mixture may be accomplished using any suitable manner, such as by direct steam injection or by using a vessel fitted with a heat exchanger. Following the addition of the last ingredient, the mixture is heated to a temperature range of from 50°F to 212°F. Temperatures outside this range are acceptable, but may be commercially impractical without use of other processing aids. When heated to the appropriate temperature, the material will typically be in the form of a thick liquid. The thick liquid is filled into cans. A lid is applied, and the container is hermetically sealed. The sealed can is then placed into conventional equipment designed to sterilize the contents. This is usually accomplished by heating to temperatures of greater than 230°F for an appropriate time, which is dependent on the temperature used and the composition.

For wet foods, the non-replicating probiotic micro-organisms can be incorporated into the wet food composition along with a carrier such as an alcohol composition (i.e., propylene glycol or dipropylene glycol), a cyclodextrin, a maltodextrin, or a starch. Alternatively, the non-replicating probiotic micro-organisms can be mixed into the dry materials prior to forming the wet food composition.

Treats can be prepared by an extrusion or baking process similar to those described above for dry food. Other processes also may be used to either coat the flavoring composition on the exterior of existing treat forms, or inject it into an existing treat form.

Animal toys are typically prepared by coating any existing toy with a flavoring composition having the non-replicating probiotic micro-organisms mixed therein.

The amount of non-replicating probiotic micro-organisms in the method for preparing a pet food composition corresponds to 10⁶ to 10¹² cfu per serving.

Obviously, non-replicating micro-organisms do not form colonies, consequently, this term is to be understood as the amount of non replicating micro-organisms that is obtained from 10⁴ and 10¹² cfu/g replicating bacteria. This includes micro-organisms that are inactivated, non-viable or dead or present as fragments such as DNA or cell wall or cytoplasmic compounds. In other words, the quantity of micro-organisms which the composition contains is expressed in terms of the colony forming ability (cfu) of that quantity of micro-organisms as if all the micro-organisms were alive irrespective of whether they are, in fact, non replicating, such as inactivated or dead, fragmented or a mixture of any or all of these states.

The pet food composition may also comprise prebiotics.

"Prebiotic" means food substances that promote the growth of probiotics in the intestines. They are not broken down in the stomach and/or upper intestine or absorbed in the GI tract of the person ingesting them, but they are fermented by the gastrointestinal microflora and/or by probiotics. Prebiotics are for example defined by Glenn R. Gibson and Marcel B. Roberfroid, Dietary Modulation of the Human Colonic Microbiota: Introducing the Concept of Prebiotics, J. Nutr. 1995 125: 1401-1412.

The prebiotics that may be used are not particularly limited and include all food substances that promote the growth of probiotics in the intestines. Preferably, they may be selected from the group consisting of oligosaccharides, optionally containing fructose, galactose, mannose; dietary fibers, in particular soluble fibers, soy fibers; inulin; or mixtures thereof. Preferred prebiotics are fructo-oligosaccharides (FOS), galacto-oligosaccharides (IOS), isomalto-oligosaccharides, xylo-oligosaccharides, oligosaccharides of soy, glycosylsucrose (GS), lactosucrose (LS), lactulose (LA), palatinose-oligosaccharides (PAO), malto-oligosaccharides (MOS), gums and/or hydrolysates thereof, pectins and/or hydrolysates thereof.

Typical examples of prebiotics are oligofructose and inulin.

The quantity of prebiotics in the pet food composition depends on their capacity to promote the development of lactic acid bacteria. As a general rule, the composition may contain from 0.1 to 20 % of such prebiotics (by weight relative to the dry matter content).

Disclosed is a pet food composition that comprises an amount of non-replicating probiotics corresponding to an amount of at least 10³ cfu per g of prebiotic, preferably 10⁴ to 10⁷ cfu/g of prebiotic, for example.

The inventors were surprised to see that, e.g., in terms of an immune boosting effect and/or in terms of an anti-inflammatory effect non-replicating probiotic microorganisms may even be more effective than replicating probiotic microorganisms.

This is surprising since probiotics are often defined as "live micro-organisms that when administered in adequate amounts confer health benefits to the host" (FAO/WHO Guidelines). The vast majority of published literature deals with live probiotics. In addition, several studies investigated the health benefits delivered by non-replicating bacteria and most of them indicated that inactivation of probiotics, e.g. by heat treatment, leads to a loss of their purported health benefit (Rachmilewitz, D., et al., 2004, Gastroenterology 126:520-528; Castagliuolo, et al., 2005, FEMS Immunol.Med.Microbiol. 43:197-204; Gill, H. S. and K. J. Rutherfurd, 2001,Br.J.Nutr. 86:285-289; Kaila, M., et al., 1995, Arch.Dis.Child 72:51-53.). Some studies showed that killed probiotics may retain some health effects (Rachmilewitz, D., et al., 2004, Gastroenterology 126:520-528; Gill, H. S. and K. J. Rutherfurd, 2001,Br.J.Nutr. 86:285-289), but clearly, living probiotics were regarded in the art so far as more performing.

"Non-replicating" probiotic micro-organisms include probiotic bacteria which have been heat treated. This includes micro-organisms that are inactivated, dead, non-viable and/or present as fragments such as DNA, metabolites, cytoplasmic compounds, and/or cell wall materials.

"Non-replicating" means that no viable cells and/or colony forming units can be detected by classical plating methods. Such classical plating methods are summarized in the microbiology book: James Monroe Jay, Martin J. Loessner, David A. Golden. 2005. Modern food microbiology. 7th edition, Springer Science, New York, N.Y. 790 p. Typically, the absence of viable cells can be shown as follows: no visible colony on agar plates or no increasing turbidity in liquid growth medium after inoculation with different concentrations of bacterial preparations ('non replicating' samples) and incubation under appropriate conditions (aerobic and/or anaerobic atmosphere for at least 24h).

Probiotics are defined as "Microbial cell preparations or components of microbial cells with a beneficial effect on the health or well-being of the host." (Salminen S, Ouwehand A. Benno Y. et al "Probiotics: how should they be defined" Trends Food Sci. Technol. 1999:10 107-10).

The compositions may comprise probiotic micro-organisms and/or non-replicating probiotic micro-organisms in an amount sufficient to at least partially produce a health benefit. An amount adequate to accomplish this is defined as "a therapeutically effective dose". Amounts effective for this purpose will depend on a number of factors known to those of skill in the art such as the weight and general health state of the animal, and on the effect of the food matrix.

In prophylactic applications, compositions are administered to a consumer susceptible to or otherwise at risk of a disorder in an amount that is sufficient to at least partially reduce the risk of developing that disorder. Such an amount is defined to be "a prophylactic effective dose". Again, the precise amounts depend on a number of factors such as the animal's state of health and weight, and on the effect of the food matrix.

Those skilled in the art will be able to adjust the therapeutically effective dose and/or the prophylactic effective dose appropriately.

In general the composition can contain non-replicating probiotic micro-organisms in a therapeutically effective dose and/or in a prophylactic effective dose.

For example, the therapeutically effective dose and/or the prophylactic effective dose may be in the range of 0,005 mg - 1000 mg non-replicating, probiotic micro-organisms per daily dose.

Discloses is that the non-replicating micro-organisms may be present in an amount equivalent to between 10⁴ to 10⁹ cfu/g of dry composition, even more preferably in an amount equivalent to between 10⁵ and 10⁹ cfu/g of dry composition.

The probiotics may be rendered non-replicating by any method that is known in the art.

The technologies available today to render probiotic strains non-replicating are usually heat-treatment, γ-irradiation, UV light or the use of chemical agents (formalin, paraformaldehyde).

In terms of numerical amounts, e.g., "short-time high temperature" treated non-replicating micro-organisms may be present in the composition in an amount corresponding to between 10⁴ and 10¹² equivalent cfu/g of the dry composition.

It would be preferred to use a technique to render probiotics non-replicating that is relatively easy to apply under industrial circumstances in the food industry.

Most products on the market today that contain probiotics are heat treated during their production. It would hence be convenient, to be able to heat treat probiotics either together with the produced product or at least in a similar way, while the probiotics retain or improve their beneficial properties or even gain a new beneficial property for the consumer.

However, inactivation of probiotic micro-organisms by heat treatments is associated in the literature generally with an at least partial loss of probiotic activity.

The present inventors have now surprisingly found, that rendering probiotic micro-organisms non-replicating, e.g., by heat treatment, does not result in the loss of probiotic health benefits, but - to the contrary - may enhance existing health benefits and even generate new health benefits.

Hence, a pet food composition is disclosed wherein the non-replicating probiotic microorganisms were rendered non-replicating by a heat-treatment.

Such a heat treatment may be carried out at at least 71.5 °C for at least 1 second.

Long-term heat treatments or short-term heat treatments may be used.

In industrial scales today usually short term heat treatments, such as UHT-like heat treatments are preferred. This kind of heat treatment reduces bacterial loads, and reduces the processing time, thereby reducing the spoiling of nutrients.

The inventors demonstrate for the first time that probiotic micro-organisms, heat treated at high temperatures for short times exhibit anti-inflammatory immune profiles regardless of their initial properties. In particular either a new anti-inflammatory profile is developed or an existing anti-inflammatory profile is enhanced by this heat treatment.

It is therefore now possible to generate non-replicating probiotic micro-organisms with anti-inflammatory profiles by using specific heat treatment parameters that correspond to typical industrially applicable heat treatments, even if live counterparts are not anti-inflammatory strains.

The high temperature treatment may be a high temperature/short time (HTST) treatment or a ultra-high temperature (UHT) treatment.

The micro-organisms may be subjected to a high temperature treatment at 120 - 140°C, for a short term of 1-30 seconds.

This high temperature treatment renders the micro-organisms at least in part non-replicating.

The high temperature treatment may be carried out at normal atmospheric pressure but may be also carried out under high pressure. Typical pressure ranges are form 1 to 50 bar, preferably from 1-10 bar, even more preferred from 2 to 5 bar. Obviously, it is preferred if the probiotics are heat treated in a medium that is either liquid or solid, when the heat is applied. An ideal pressure to be applied will therefore depend on the nature of the composition which the micro-organisms are provided in and on the temperature used.

The high temperature treatment may be carried out in the temperature range of 120-140 °C.

The high temperature treatment may be carried out for a short term of 1-30 seconds, preferably 5-15 seconds.

This given time frame refers to the time the probiotic micro-organisms are subjected to the given temperature. Note, that depending on the nature and amount of the composition the micro-organisms are provided in and depending on the architecture of the heating apparatus used, the time of heat application may differ.

The composition and/or the micro-organisms are treated by a high temperature short time (HTST) treatment or a ultra high temperature (UHT) treatment.

A UHT treatment is Ultra-high temperature processing or a ultra-heat treatment (both abbreviated UHT) involving the at least partial sterilization of a composition by heating it for a short time, around 1-10 seconds, at a temperature exceeding 135°C (275°F), which is the temperature required to kill bacterial spores in milk. For example, processing milk in this way using temperatures exceeding 135°C permits a decrease of bacterial load in the necessary holding time (to 2-5 s) enabling a continuous flow operation.

There are two main types of UHT systems: the direct and indirect systems. In the direct system, products are treated by steam injection or steam infusion, whereas in the indirect system, products are heat treated using plate heat exchanger, tubular heat exchanger or scraped surface heat exchanger. Combinations of UHT systems may be applied at any step or at multiple steps in the process of product preparation.

A HTST treatment is defined as follows (High Temperature/Short Time): Pasteurization method designed to achieve a 5-log reduction, killing 99,9999% of the number of viable micro-organisms in milk. This is considered adequate for destroying almost all yeasts, molds and common spoilage bacteria and also to ensure adequate destruction of common pathogenic heat resistant organisms. In the HTST process milk is heated to 71.7oC (161°F) for 15-20 seconds.

Disclosed is that flash pasteurization is a method of heat pasteurization of perishable beverages like fruit and vegetable juices, beer and dairy products. It is done prior to filling into containers in order to kill spoilage micro-organisms, to make the products safer and extend their shelf life. The liquid moves in controlled continuous flow while subjected to temperatures of 71.5°C (160°F) to 74°C (165°F) for 15 to 30 seconds.

Disclosed is that the term "short time high temperature treatment" shall include high-temperature short time (HTST) treatments, UHT treatments, and flash pasteurization, for example.

Since such a heat treatment provides non-replicating probiotics with an improved anti-inflammatory profile; the composition may be for use in the prevention or treatment of inflammatory disorders.

The inflammatory disorders that can be treated or prevented by the composition are not particularly limited. For example, they may be selected from the group consisting of acute inflammations such as sepsis; burns; and chronic inflammation, such as inflammatory bowel disease, e.g., Crohn's disease, ulcerative colitis, pouchitis; necrotizing enterocolitis; skin inflammation, such as UV or chemical-induced skin inflammation, eczema, reactive skin; irritable bowel syndrome; eye inflammation; allergy, asthma; and combinations thereof.

Disclosed are heat treatments which render the probiotic micro-organisms non-replicating, such a heat treatment may be carried out in the temperature range of 70-150 °C for 3 minutes - 2 hours, preferably in the range of 80-140°C from 5 minutes - 40 minutes.

While the prior art generally teaches that bacteria rendered non-replicating by long-term heat-treatments are usually less efficient than live cells in terms of exerting their probiotic properties, it was demonstrated that long-term heat-treated probiotics are superior in stimulating the immune system compared to their live counterparts.

Disclosed is also a composition comprising probiotic micro-organisms that were rendered non-replicating by a heat treatment at at least 70 °C for at least 3 minutes.

The immune boosting effects of non-replicating probiotics were confirmed by *in vitro* immunoprofiling. The *in vitro* model used uses cytokine profiling from human Peripheral Blood Mononuclear Cells (PBMCs) and is well accepted in the art as standard model for tests of immunomodulating compounds (Schultz et al., 2003, Journal of Dairy Research 70, 165-173;Taylor et al., 2006, Clinical and Experimental Allergy, 36, 1227-1235; Kekkonen et al., 2008, World Journal of Gastroenterology, 14, 1192-1203)

The *in vitro* PBMC assay has been used by several authors/research teams for example to classify probiotics according to their immune profile, i.e. their anti- or pro-inflammatory characteristics (Kekkonen et al., 2008, World Journal of Gastroenterology, 14, 1192-1203). For example, this assay has been shown to allow prediction of an anti-inflammatory effect of probiotic candidates in mouse models of intestinal colitis (Foligne, B., et al., 2007, World J.Gastroenterol. 13:236-243). Moreover, this assay is regularly used as read-out in clinical trials and was shown to lead to results coherent with the clinical outcomes (Schultz et al., 2003, Journal of Dairy Research 70, 165-173; Taylor et al., 2006, Clinical and Experimental Allergy, 36, 1227-1235).

Allergic diseases have steadily increased over the past decades and they are currently considered as epidemics by WHO. In a general way, allergy is considered to result from an imbalance between the Th1 and Th2 responses of the immune system leading to a strong bias towards the production of Th2 mediators. Therefore, allergy can be mitigated, down-regulated or prevented by restoring an appropriate balance between the Th1 and Th2 arms of the immune system. This implies the necessity to reduce the Th2 responses or to enhance, at least transiently, the Th1 responses. The latter would be characteristic of an immune boost response, often accompanied by for example higher levels of IFNγ, TNF-α and IL-12. (Kekkonen et al., 2008, World Journal of Gastroenterology, 14, 1192-1203; Viljanen M. et al., 2005, Allergy, 60, 494-500)

The pet food composition allows it hence to treat or prevent disorders that are related to a compromised immune defence.

Consequently, the disorders linked to a compromised immune defence that can be treated or prevented are not particularly limited.

For example, they may be selected from the group consisting of infections, in particular bacterial, viral, fungal and/or parasite infections; phagocyte deficiencies; low to severe immunodepression levels such as those induced by stress or immunodepressive drugs, chemotherapy or radiotherapy; natural states of less immunocompetent immune systems such as those of the neonates; allergies; and combinations thereof.

The pet food composition allows it also to enhance an animal's response to vaccines, in particular to oral vaccines.

At least 90 %, preferably, at least 95 %, more preferably at least 98 %, most preferably at least 99 %, ideally at least 99.9 %, most ideally all of the probiotics are non-replicating.

Disclosed is that all micro-organisms are non-replicating.

Consequently, in the composition at least 90 %, preferably, at least 95 %, more preferably at least 98 %, most preferably at least 99 %, ideally at least 99.9 %, most ideally all of the probiotics may be non-replicating.

All probiotic micro-organisms may be used for the purpose of the present invention.

For example, the probiotic micro-organisms may be selected from the group consisting of bifidobacteria, lactobacilli, propionibacteria, or combinations thereof, for example *Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus reuteri, Lactobacillus rhamnosus, Lactobacillus johnsonii, Lactobacillus plantarum, Lactobacillus fermentum, Lactococcus lactis, Streptococcus thermophilus*, *Lactococcus lactis, Lactococcus diacetylactis, Lactococcus cremoris, Lactobacillus bulgaricus*, *Lactobacillus helveticus, Lactobacillus delbrueckii, Escherichia coli* and/or mixtures thereof.

The composition in accordance with the present may, for example comprise probiotic micro-organisms selected from the group consisting of Bifidobacterium longum NCC 3001 (ATCC BAA-999), Bifidobacterium longum NCC 2705 (CNCM I-2618), Bifidobacterium breve NCC 2950 (CNCM I-3865), Bifidobacterium lactis NCC 2818 (CNCM I-3446), Lactobacillus johnsonii La 1 (CNCM I-1225), Lactobacillus paracasei NCC 2461 (CNCM I-2116), Lactobacillus rhamnosus NCC 4007 (CGMCC 1.3274), Lactobacillus reuteri DSM17983, Lactobacillus reuteri ATCC 55730, Streptococcus thermophilus NCC 2059 (CNCM I-4153), Lactobacillus casei NCC 1825 (ACA-DC 6002), Escherichia coli Nissle (DSM 6601), Lactobacillus bulgaricus NCC 15 (CNCM I-1198), Lactococcus lactis NCC 2287 (CNCM I-4154), or combinations thereof.

All these strains were either deposited under the Budapest treaty and/or are commercially available.

The strains have been deposited under the Budapest treaty as follows:

| | |
|---|---|
| Bifidobacterium longum NCC 3001: | ATCC BAA-999 |
| Bifidobacterium longum NCC 2705: | CNCM I-2618 |
| Bifidobacterium breve NCC 2950 | CNCM I-3865 |
| Bifidobacterium lactis NCC 2818: | CNCM I-3446 |
| Lactobacillus paracasei NCC 2461: | CNCM I-2116 |
| Lactobacillus rhamnosus NCC 4007: | CGMCC 1.3724 |
| Streptococcus themophilus NCC 2019: | CNCM I-1422 |
| Streptococcus themophilus NCC 2059: | CNCM I-4153 |
| Lactococcus lactis NCC 2287: | CNCM I-4154 |
| Lactobacillus casei NCC 4006: | CNCM I-1518 |
| Lactobacillus casei NCC 1825: | ACA-DC 6002 |
| Lactobacillus acidophilus NCC 3009: | ATCC 700396 |
| Lactobacillus bulgaricus NCC 15: | CNCM I-1198 |
| Lactobacillus johnsonii La1 | CNCM I-1225 |
| Lactobacillus reuteri DSM17983 | DSM17983 |
| Lactobacillus reuteri ATCC55730 | ATCC55730 |
| Escherichia coli Nissle 1917: | DSM 6601 |

Strains named ATCC were deposited with the ATCC Patent Depository, 10801 University Blvd., Manassas, VA 20110, USA.

Strains named CNCM were deposited with the COLLECTION NATIONALE DE CULTURES DE MICROORGANISMES (CNCM), 25 rue du Docteur Roux, F-75724 PARIS Cedex 15, France.

Strains named CGMCC were deposited with the China General Microbiological Culture Collection Center, Institute of Microbiology, Chinese Academy of Sciences, Zhongguancun, P.O.Box2714, Beijing 100080, China.

Strains named ACA-DC were deposited with the Greek Coordinated Collections of Microorganisms, Dairy Laboratory,Department of Food Science and Technology,Agricultural University of Athens, 75, Iera odos, Botanikos, Athens, 118 55, Greece.

Strains named DSM were deposited with the DSMZ-Deutsche Sammlung von Mikroorganismen und Zellkulturen GmbH, Inhoffenstr. 7 B^, 38124 Braunschweig, GERMANY.

Those skilled in the art will understand that they can freely combine all features of the present invention described herein, without departing from the scope of the invention as disclosed.

Further advantages and features are apparent from the following Examples and Figures.
Figures 1A and B show the enhancement of the anti-inflammatory immune profiles of probiotics treated with "short-time high temperatures".
Figure 2 shows non anti-inflammatory probiotic strains that become anti-inflammatory, i.e. that exhibit pronounced anti-inflammatory immune profiles *in vitro* after being treated with "short-time high temperatures".
Figures 3A and B show probiotic strains in use in commercially available products that exhibit enhanced or new anti-inflammatory immune profiles *in vitro* after being treated with "short-time high temperatures".
Figures 4A and B show dairy starter strains (i.e. Lc1 starter strains) that exhibits enhanced or new anti-inflammatory immune profiles *in vitro* upon heat treatment at high temperatures.
Figure 5 shows a non anti-inflammatory probiotic strain that exhibits anti-inflammatory immune profiles *in vitro* after being treated with HTST treatments.
Figure 6: Principal Component Analysis on PBMC data (IL-12p40, IFN-γ, TNF-α, IL-10) generated with probiotic and dairy starter strains in their live and heat treated (140°C for 15 second) forms. Each dot represents one strain either live or heat treated identified by its NCC number or name.
Figure 7 shows IL-12p40 / IL-10 ratios of live and heat treated (85°C, 20min) strains. Overall, heat treatment at 85°C for 20 min leads to an increase of IL-12p40 / IL-10 ratios as opposed to "short-time high temperature" treatments of the present invention (Figures 1, 2, 3, 4 and 5).
Figure 8 shows the enhancement of *in vitro* cytokine secretion from human PBMCs stimulated with heat treated bacteria.
Figure 9 shows the percentage of diarrhea intensity observed in OVA-sensitized mice challenged with saline (negative control), OVA-sensitized mice challenged with OVA (positive control) and OVA-sensitized mice challenged with OVA and treated with heat-treated or live *Bifidobacterium breve* NCC2950. Results are displayed as the percentage of diarrhea intensity (Mean ± SEM calculated from 4 independent experiments) with 100 % of diarrhea intensity corresponding to the symptoms developed in the positive control (sensitized and challenged by the allergen) group.

### Example 1:

### Methodology

### Bacterial preparations:

The health benefits delivered by live probiotics on the host immune system are generally considered to be strain specific. Probiotics inducing high levels of IL-10 and/or inducing low levels of pro-inflammatory cytokines *in vitro* (PBMC assay) have been shown to be potent anti-inflammatory strains *in vivo* (Foligné, B., et al., 2007, World J.Gastroenterol. 13:236-243).

Several probiotic strains were used to investigate the anti-inflammatory properties of heat treated probiotics. These were *Bifidobacterium longum* NCC 3001, *Bifidobacterium longum* NCC 2705, *Bifidobacterium breve* NCC 2950, *Bifidobacterium lactis* NCC 2818, *Lactobacillus paracasei* NCC 2461, *Lactobacillus rhamnosus* NCC 4007, *Lactobacillus casei* NCC 4006, *Lactobacillus acidophilus* NCC 3009, *Lactobacillus casei* ACA-DC 6002 (NCC 1825), and *Escherichia coli* Nissle. Several starter culture strains including some strains commercially used to produce Nestlé Lc1 fermented products were also tested: *Streptococcus thermophilus* NCC 2019, *Streptococcus thermophilus* NCC 2059, *Lactobacillus bulgaricus* NCC 15 and *Lactococcus lactis* NCC 2287.

Bacterial cells were cultivated in conditions optimized for each strain in 5-15L bioreactors. All typical bacterial growth media are usable. Such media are known to those skilled in the art. When pH was adjusted to 5.5, 30% base solution (either NaOH or Ca(OH)₂) was added continuously. When adequate, anaerobic conditions were maintained by gassing headspace with CO₂. *E. coli* was cultivated under standard aerobic conditions.

Bacterial cells were collected by centrifugation (5,000 x g, 4°C) and re-suspended in phosphate buffer saline (PBS) in adequate volumes in order to reach a final concentration of around 10⁹ -10¹⁰ cfu/ml. Part of the preparation was frozen at -80°C with 15% glycerol. Another part of the cells was heat treated by:
- Ultra High Temperature: 140°C for 15 sec; by indirect steam injection.
- High Temperature Short Time (HTST): 74°C, 90°C and 120°C for 15 sec by indirect steam injection
- Long Time Low Temperature (85°C, 20 min) in water bath

Upon heat treatment, samples were kept frozen at -80°C until use.

### In vitro immunoprofiling of bacterial preparations:

The immune profiles of live and heat treated bacterial preparations (i.e. the capacity to induce secretion of specific cytokines from human blood cells *in vitro*) were assessed. Human peripheral blood mononuclear cells (PBMCs) were isolated from blood filters. After separation by cell density gradient, mononuclear cells were collected and washed twice with Hank's balanced salt solution. Cells were then resuspended in Iscove's Modified Dulbecco's Medium (IMDM, Sigma) supplemented with 10% foetal calf serum (Bioconcept, Paris, france), 1% L-glutamine (Sigma), 1% penicillin/streptomycin (Sigma) and 0.1% gentamycin (Sigma). PBMCs (7x10⁵ cells/well) were then incubated with live and heat treated bacteria (equivalent 7x10⁶ cfu/well) in 48 well plates for 36h. The effects of live and heat treated bacteria were tested on PBMCs from 8 individual donors splitted into two separated experiments. After 36h incubation, culture plates were frozen and kept at -20°C until cytokine measurement. Cytokine profiling was performed in parallel (i.e. in the same experiment on the same batch of PBMCs) for live bacteria and their heat-treated counterparts.

Levels of cytokines (IFN-γ, IL-12p40, TNF-α and IL-10) in cell culture supernatants after 36h incubation were determined by ELISA (R&D DuoSet Human IL-10, BD OptEIA Human IL12p40, BD OptEIA Human TNFα, BD OptEIA Human IFN-γ) following manufacturer's instructions. IFN-γ, IL-12p40 and TNF-α are pro-inflammatory cytokines, whereas IL-10 is a potent anti-inflammatory mediator. Results are expressed as means (pg/ml) +/- SEM of 4 individual donors and are representative of two individual experiments performed with 4 donors each. The ratio IL-12p40 / IL-10 is calculated for each strain as a predictive value of *in vivo* anti-inflammatory effect (Foligné, B., et al., 2007, World J.Gastroenterol. 13:236-243).

Numerical cytokine values (pg/ml) determined by ELISA (see above) for each strain were transferred into BioNumerics v5.10 software (Applied Maths, Sint-Martens-Latem, Belgium). A Principal Component Analysis (PCA, dimensioning technique) was performed on this set of data. Subtraction of the averages over the characters and division by the variances over the characters were included in this analysis.

### Results

Anti-inflammatory profiles generated by Ultra High Temperature (UHT) / High Temperature Short Time (HTST)-like treatments

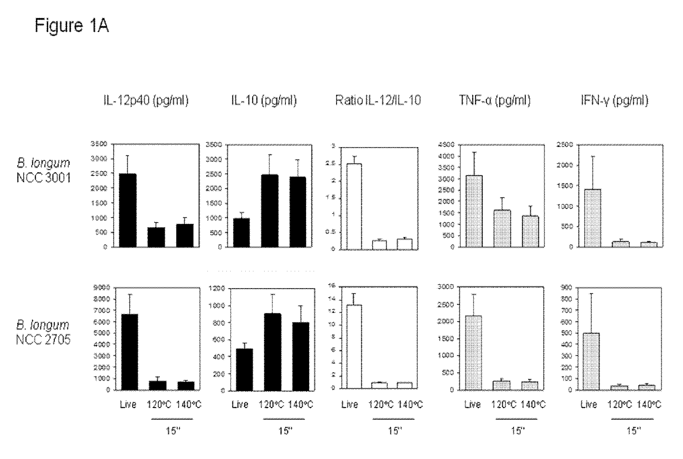

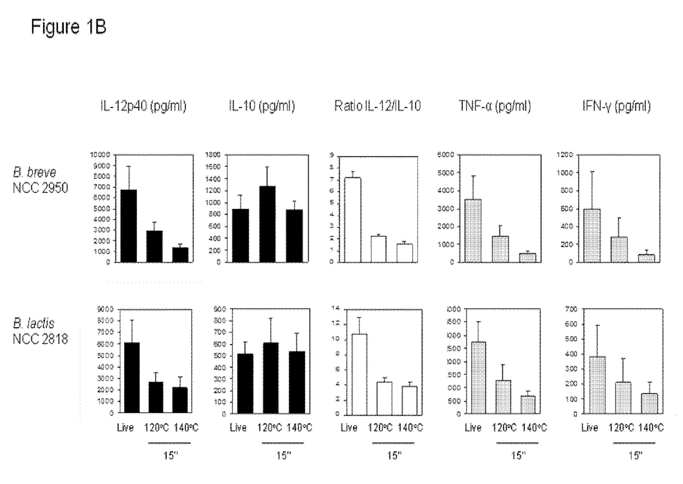

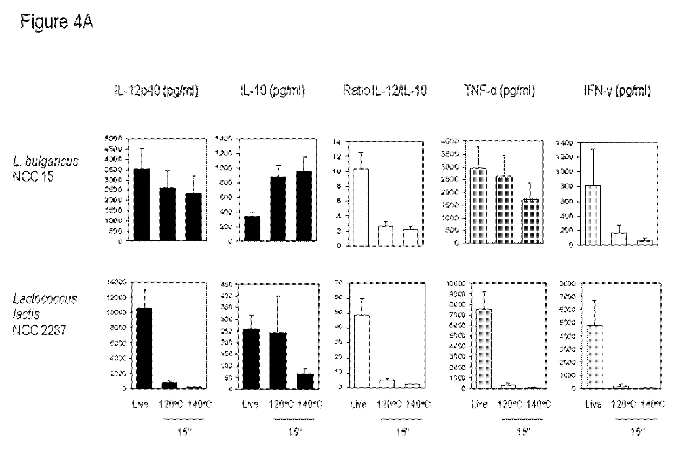

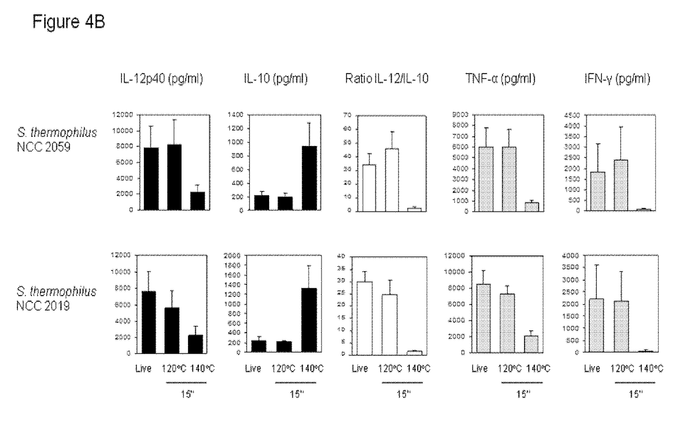

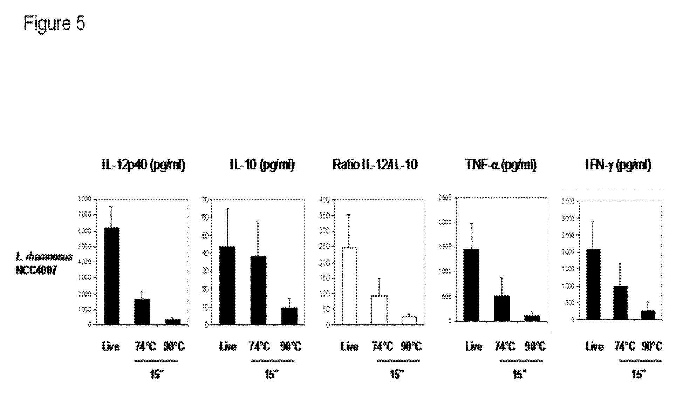

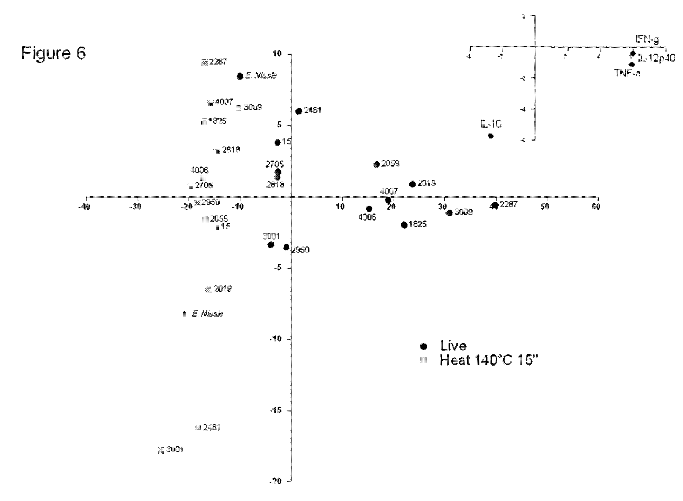

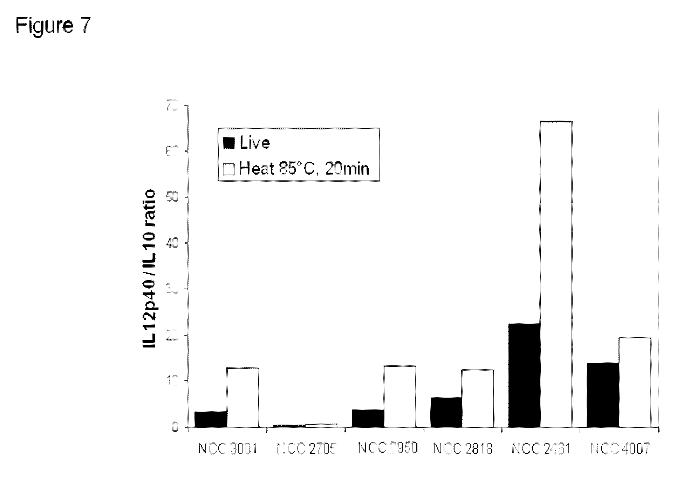

The probiotic strains under investigation were submitted to a series of heat treatments (Ultra High Temperature (UHT), High Temperature Short Time (HTST) and 85°C for 20 min) and their immune profiles were compared to those of live cells *in vitro.* Live micro-organisms (probiotics and/or dairy starter cultures) induced different levels of cytokine production when incubated with human PBMC (Figures 1, 2, 3, 4 and 5). Heat treatment of these micro-organisms modified the levels of cytokines produced by PBMC in a temperature dependent manner. "Short-time high temperature" treatments (120°C or 140°C for 15") generated non replicating bacteria with anti-inflammatory immune profiles (Figures 1, 2, 3 and 4). Indeed, UHT-like treated strains (140°C, 15 sec) induced less pro-inflammatory cytokines (TNF-α, IFN-γ, IL-12p40) while maintaining or inducing additional IL-10 production (compared to live counterparts). The resulting IL-12p40 / IL-10 ratios were lower for any UHT-like treated strains compared to live cells (Figures 1, 2, 3 and 4). This observation was also valid for bacteria treated by HTST-like treatments, i.e. submitted to 120°C for 15 sec (Figures 1, 2, 3 and 4), or 74°C and 90°C for 15 sec (Figure 5). Heat treatments (UHT-like or HTST-like treatments) had a similar effect on *in vitro* immune profiles of probiotic strains (Figures 1, 2, 3 and 5) and dairy starter cultures (Figure 4). Principal Component Analysis on PBMC data generated with live and heat treated (140°C, 15") probiotic and dairy starter strains revealed that live strains are spread all along the x axis, illustrating that strains exhibit very different immune profiles *in vitro,* from low (left side) to high (right side) inducers of pro-inflammatory cytokines. Heat treated strains cluster on the left side of the graph, showing that pro-inflammatory cytokines are much less induced by heat treated strains (Figure 6). By contrast, bacteria heat treated at 85°C for 20 min induced more pro-inflammatory cytokines and less IL-10 than live cells resulting in higher IL-12p40 / IL-10 ratios (Figure 7).

Anti-inflammatory profiles are enhanced or generated by UHT-like and HTST-like treatments.

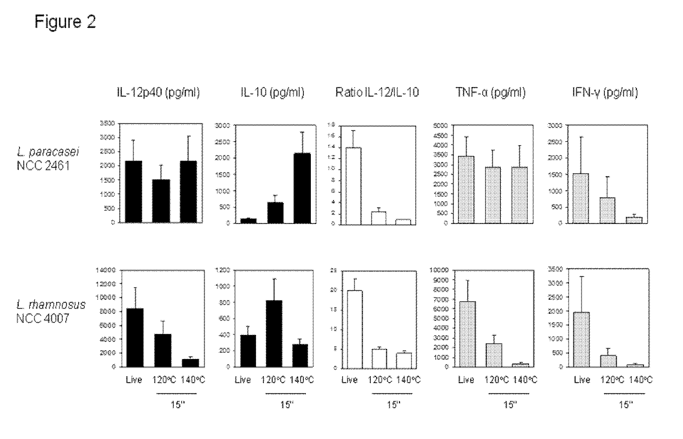

UHT and HTST treated strains exhibit anti-inflammatory profiles regardless of their respective initial immune profiles (live cells). Probiotic strains known to be anti-inflammatory *in vivo* and exhibiting anti-inflammatory profiles *in vitro* (*B. longum* NCC 3001, *B. longum* NCC 2705, *B. breve* NCC 2950, *B. lactis* NCC 2818) were shown to exhibit enhanced anti-inflammatory profiles *in vitro* after "short-time high temperature" treatments. As shown in Figure 1, the IL-12p40 / IL-10 ratios of UHT-like treated *Bifidobacterium* strains were lower than those from the live counterparts, thus showing improved anti-inflammatory profiles of UHT-like treated samples. More strikingly, the generation of anti-inflammatory profiles by UHT-like and HTST-like treatments was also confirmed for non anti-inflammatory live strains. Both live *L. rhamnosus* NCC 4007 and *L. paracasei* NCC 2461 exhibit high IL-12p40 / IL-10 ratios *in vitro* (Figures 2 and 5). The two live strains were shown to be not protective against TNBS-induced colitis in mice. The IL-12p40 / IL-10 ratios induced by *L. rhamnosus* NCC 4007 and *L. paracasei* NCC 2461 were dramatically reduced after "short-time high temperature" treatments (UHT or HTST) reaching levels as low as those obtained with *Bifidobacterium* strains. These low IL-12p40 / IL-10 ratios are due to low levels of IL-12p40 production combined with no change (*L. rhamnosus* NCC 4007) or a dramatic induction of IL-10 secretion (*L. paracasei* NCC 2461) (Figure 2).

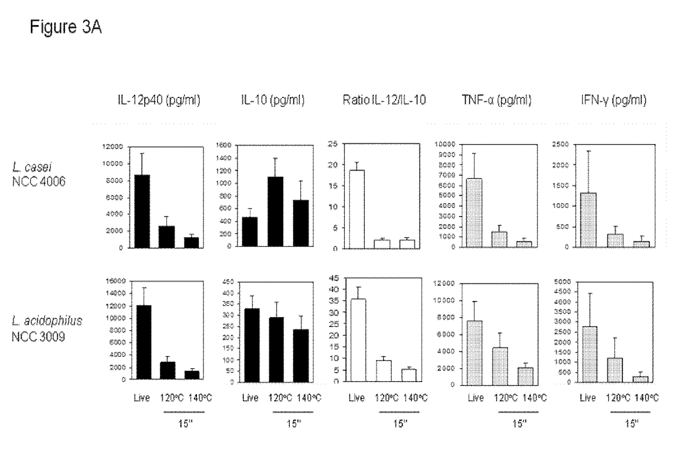

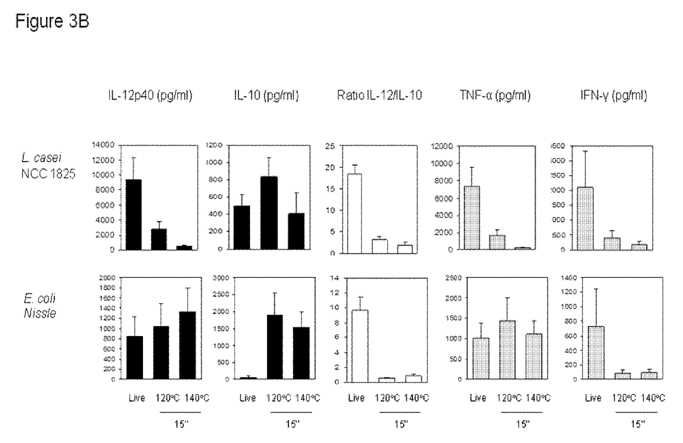

As a consequence:
- Anti-inflammatory profiles of live micro-organisms can be enhanced by UHT-like and HTST-like heat treatments (for instance *B. longum* NCC 2705, *B. longum* NCC 3001, *B. breve* NCC 2950, *B. lactis* NCC 2818)
- Anti-inflammatory profiles can be generated from non anti-inflammatory live micro-organisms (for example *L. rhamnosus* NCC 4007, *L. paracasei* NCC 2461, dairy starters *S. thermophilus* NCC 2019) by UHT-like and HTST-like heat treatments.
- Anti-inflammatory profiles were also demonstrated for strains isolated from commercially available products (Figures 3A & B) including a probiotic *E. coli* strain.

The impact of UHT/HTST-like treatments was similar for all tested probiotics and dairy starters, for example lactobacilli, bifidobacteria and streptococci.

UHT/HTST-like treatments were applied to several lactobacilli, bifidobacteria and streptococci exhibiting different *in vitro* immune profiles. All the strains induced less pro-inflammatory cytokines after UHT/HTST-like treatments than their live counterparts (Figures 1, 2, 3, 4, 5 and 6) demonstrating that the effect of UHT/HTST-like treatments on the immune properties of the resulting non replicating bacteria can be generalized to all probiotics, in particular to lactobacilli and bifidobacteria and specific *E. coli* strains and to all dairy starter cultures in particular to streptococci, lactococci and lactobacilli.

### Example 2 (comparative example):

### Methodology

### Bacterial preparations:

Five probiotic strains were used to investigate the immune boosting properties of non-replicating probiotics: 3 bifidobacteria (*B. longum* NCC3001, *B. lactis* NCC2818, *B. breve* NCC2950) and 2 lactobacilli (*L. paracasei* NCC2461, *L. rhamnosus* NCC4007).

Bacterial cells were grown on MRS in batch fermentation at 37°C for 16-18h without pH control. Bacterial cells were spun down (5,000 x g, 4°C) and resuspended in phosphate buffer saline prior to be diluted in saline water in order to reach a final concentration of around 10E10 cfu/ml. *B. longum* NCC3001, *B. lactis* NCC2818, *L. paracasei* NCC2461, *L. rhamnosus* NCC4007 were heat treated at 85°C for 20 min in a water bath. *B. breve* NCC2950 was heat treated at 90°C for 30 minutes in a water bath. Heat treated bacterial suspensions were aliquoted and kept frozen at -80°C until use. Live bacteria were stored at - 80°C in PBS-glycerol 15% until use.

### In vitro immunoprofiling of bacterial preparations

The immune profiles of live and heat treated bacterial preparations (i.e. the capacity to induce secretion of specific cytokines from human blood cells *in vitro)* were assessed. Human peripheral blood mononuclear cells (PBMCs) were isolated from blood filters. After separation by cell density gradient, mononuclear cells were collected and washed twice with Hank's balanced salt solution. Cells were then resuspended in Iscove's Modified Dulbecco's Medium (IMDM, Sigma) supplemented with 10% foetal calf serum (Bioconcept, Paris, france), 1% L-glutamine (Sigma), 1% penicillin/streptomycin (Sigma) and 0.1% gentamycin (Sigma). PBMCs (7x10⁵ cells/well) were then incubated with live and heat treated bacteria (equivalent 7x10⁶ cfu/well) in 48 well plates for 36h. The effects of live and heat treated bacteria were tested on PBMCs from 8 individual donors splitted into two separate experiments. After 36h incubation, culture plates were frozen and kept at -20°C until cytokine measurement. Cytokine profiling was performed in parallel (i.e. in the same experiment on the same batch of PBMCs) for live bacteria and their heat-treated counterparts.

Levels of cytokines (IFN-γ, IL-12p40, TNF-α and IL-10) in cell culture supernatants after 36h incubation were determined by ELISA (R&D DuoSet Human IL-10, BD OptEIA Human IL12p40, BD OptEIA Human TNF, BD OptEIA Human IFN-γ) following manufacturer's instructions. IFN-γ, IL-12p40 and TNF-α are pro-inflammatory cytokines, whereas IL-10 is a potent anti-inflammatory mediator. Results are expressed as means (pg/ml) +/- SEM of 4 individual donors and are representative of two individual experiments performed with 4 donors each.

### In vivo effect of live and heat treated Bifidobacterium breve NCC2950 in prevention of allergic diarrhea

A mouse model of allergic diarrhea was used to test the Th1 promoting effect of *B. breve* NCC2950 (Brandt E.B et al. JCI 2003; 112 (11) : 1666-1667). Following sensitization (2 intraperitoneal injections of Ovalbumin (OVA) and aluminium potassium sulphate at an interval of 14 days; days 0 and 14) male Balb/c mice were orally challenged with OVA for 6 times (days 27, 29, 32, 34, 36, 39) resulting in transient clinical symptoms (diarrhea) and changes of immune parameters (plasma concentration of total IgE, OVA specific IgE, mouse mast cell protease 1, i.e MMCP-1). *Bifidobacterium breve* NCC2950 live or heat treated at 90°C for 30min, was administered by gavage 4 days prior to OVA sensitization (days -3, -2, -1, 0 and days 11, 12, 13 and 14) and during the challenge period (days 23 to 39). A daily bacterial dose of around 10⁹ colony forming units (cfu) or equivalent cfu/mouse was used.

### Results

### Induction of secretion of 'pro-inflammatory' cytokines after heat treatment

The ability of heat treated bacterial strains to stimulate cytokine secretion by human peripheral blood mononuclear cells (PBMCs) was assessed *in vitro.* The immune profiles based on four cytokines upon stimulation of PBMCs by heat treated bacteria were compared to that induced by live bacterial cells in the same *in vitro* assay.

The heat treated preparations were plated and assessed for the absence of any viable counts. Heat treated bacterial preparations did not produce colonies after plating.

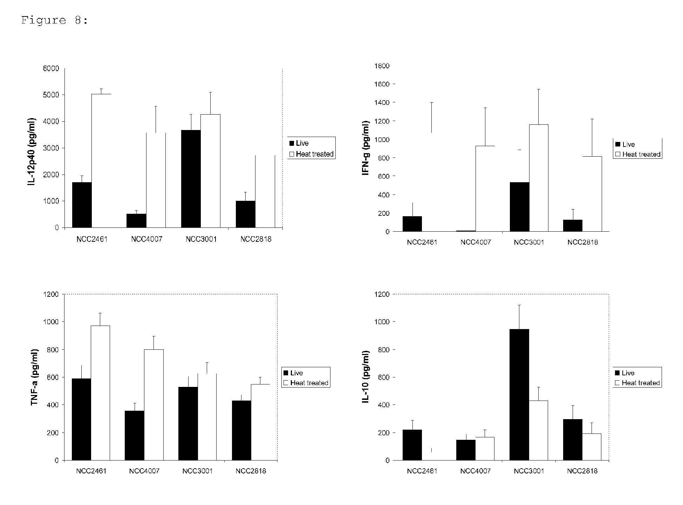

Live probiotics induced different and strain dependent levels of cytokine production when incubated with human PBMCs (Figure 8). Heat treatment of probiotics modified the levels of cytokines produced by PBMCs as compared to their live counterparts. Heat treated bacteria induced more pro-inflammatory cytokines (TNF-α, IFN-γ, IL-12p40) than their live counterparts do. By contrast heat treated bacteria induced similar or lower amounts of IL-10 compared to live cells (Figure 8). These data show that heat treated bacteria are more able to stimulate the immune system than their live counterparts and therefore are more able to boost weakened immune defences. In other words the *in vitro* data illustrate an enhanced immune boost effect of bacterial strains after heat treatment.

In order to illustrate the enhanced effect of heat-treated *B. breve* NCC2950 (compared to live cells) on the immune system, both live and heat treated *B. breve* NCC2950 (strain A) were tested in an animal model of allergic diarrhea.

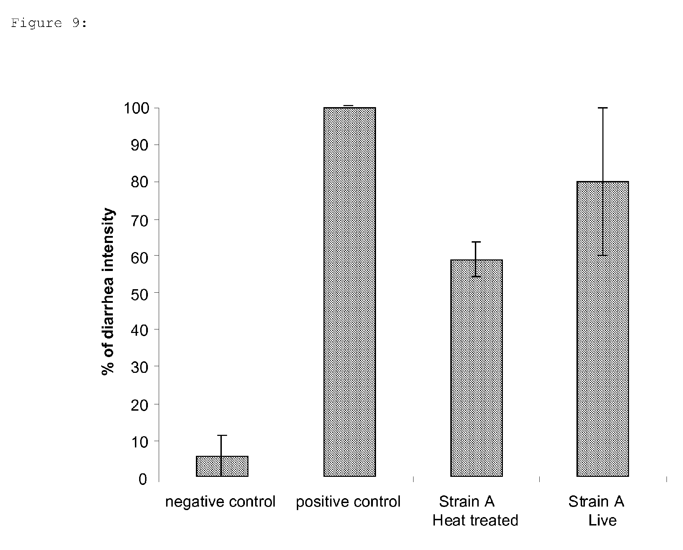

As compared to the positive control group, the intensity of diarrhea was significantly and consistently decreased after treatment with heat treated *B. breve* NCC2950 (41.1 % ± 4.8) whereas the intensity of diarrhea was lowered by only 20 ± 28.3 % after treatment with live *B. breve* NCC2950. These results demonstrate that heat-*treated B.* breve NCC2950 exhibits an enhanced protective effect against allergic diarrhea than its live counterpart (Figure 9).

As a consequence, the ability of probiotics to enhance the immune defences was shown to be improved after heat treatment.

### Further Example:

The following pet food composition may be prepared using standard techniques as described in this patent application:

| **Ingredient** | **g/100g** |
|---|---|
| Fat | 15 |
| Protein | 29 |
| Carbohydrates | 46 |
| Dietary fiber | 7 |
| Nutritional balancing agents | 2 |
| Short term heat treated Lactobacillus johnsonii La1 | 1 |

## Claims

1. A method for preparing a pet food composition comprising non-replicating probiotic micro-organisms in an amount corresponding to 10⁶ to 10¹² cfu per serving said method comprising treating the probiotic micro-organisms with a high temperature/ short time (HTST) treatment at a temperature of 120-140°C for 1-30 seconds or a ultra-high temperature (UHT) treatment at a temperature exceeding 135°C for 1-10 seconds, and thus rendering at least 90% of the micro-organisms non-replicating.

2. A method in accordance with claim 1 wherein said pet food composition is comprising from 4 to 40 weight-% dry weight fat, from 12 to 70 weight-% dry weight carbohydrates, and from 12 to 50 weight-% dry weight proteins.

3. A method in accordance with claim 2 wherein said pet food composition is comprising from 10 to 20 weight-% dry weight fat, from 30 to 60 weight-% dry weight carbohydrates, and from 20 to 35 weight-% dry weight proteins.

4. A method in accordance with one of the preceding claims, wherein said pet food composition is further comprising from 0.5 to 40 weight-% dry weight, preferably from 0.5 to 30 weight-% dry weight, more preferably from 1 to 20 weight-% dry weight, most preferably from 1 to 10 weight-% dry weight dietary fiber.

5. A method in accordance with one of the preceding claims, wherein the pet food composition is selected from the group consisting of pet foods, nutritional diets for pets, supplements for pets, treats for pets, and food toys for pets such as chewable and consumable toys.

6. A method in accordance with one of the preceding claims, wherein said pet food composition is further comprising prebiotics, for example oligofructose and inulin.

7. A method in accordance with one of the preceding claims wherein in said pet food composition at least 95 %, preferably at least 98 %, more preferably at least 99 %, most at least 99.9 %, ideally all of the probiotics are non-replicating.

8. A method in accordance with one of the preceding claims wherein the probiotic micro-organisms are selected from the group consisting of bifidobacteria, lactobacilli, propionibacteria, or combinations thereof, for example Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus reuteri, Lactobacillus rhamnosus, Lactobacillus johnsonii, Lactobacillus plantarum, Lactobacillus fermentum, Lactococcus lactis, Streptococcus thermophilics, Lactococcus lactis, Lactococcus diacetylactis, Lactococcus cremoris, Lactobacillus bulgaricus, Lactobacillus helveticusr Lactobacillus delbrueckii, Escherichia coli and/or mixtures thereof.

9. A method in accordance with one of the preceding claims wherein the probiotic micro-organisms are selected from the group consisting of Bifidobacterium longum NCC 3001 (ATCC BAA-999), Bifidobacterium longum NCC 2705 (CNCM 1-2618), Bifidobacterium breve NCC 2950 (CNCM 1-3865), Bifidobacterium lactis NCC 2818 (CNCM 1-3446), Lactobacillus johnsonii La 1 (CNCM I-1225), Lactobacillus paracasei NCC 2461 (CNCM I-2116), Lactobacillus rhamnosus NCC 4007 (CGMCC 1.3274), Lactobacillus reuteri DSM17983, Lactobacillus reuteri ATCC 55730, Streptococcus thermophilus NCC 2059 (CNCM 1-4153), Lactobacillus casei NCC 1825 (ACA-DC 6002), Escherichia coli Nissle (DSM 6601), Lactobacillus bulgaricus NCC 15 (CNCM 1-1198), Lactococcus lactis NCC 2287 (CNCM 1-4154), or combinations thereof.

10. A method in accordance with one of the preceding claims, wherein said pet food composition is containing 0,005 mg - 1000 mg non- replicating micro-organisms per daily dose.

## Patentansprüche

1. Verfahren zur Zubereitung einer Haustierfutterzusammensetzung mit nicht replizierenden probiotischen Mikroorganismen in einer Menge entsprechend 10⁶ bis 10¹² KBE pro Portion, wobei das Verfahren die Behandlung der probiotischen Mikroorganismen mit einer Hochtemperatur-/Kurzzeitbehandlung (HTST) bei einer Temperatur von 120 bis 140°C über 1-30 Sekunden oder einer Ultrahochtemperaturbehandlung (UHT) bei einer Temperatur oberhalb 135°C über 1 bis 10 Sekunden umfasst und somit mindestens 90% der Mikroorganismen nicht replizierend macht.

2. Verfahren gemäß Anspruch 1, wobei die Haustierfutterzusammensetzung von 4 bis 40 Gew-% Trockenmasse an Fett, von 12 bis 70 Gew-% Trockenmasse an Kohlenhydraten und von 12 bis 50 Gew-% Trockenmasse an Eiweißen enthält.

3. Verfahren gemäß Anspruch 2, wobei die Haustierfutterzusammensetzung von 10 bis 20 Gew-% Trockenmasse an Fett, von 30 bis 60 Gew-% Trockenmasse an Kohlenhydraten, und von 20 bis 35 Gew-% Trockenmasse an Eiweißen enthält.

4. Verfahren gemäß einem der vorhergehenden Ansprüche, wobei die Haustierfutterzusammensetzung weiterhin von 0,5 bis 40 Gew-% Trockenmasse, vorzugsweise von 0,5 bis 30 Gew-% Trockenmasse, weiter bevorzugt von 1 bis 20 Gew-% Trockenmasse, höchst bevorzugt von 1 bis 10 Gew-% Trockenmasse an Ballaststoffen enthält.

5. Verfahren gemäß einem der vorhergehenden Ansprüche, wobei die Haustierfutterzusammensetzung aus der Gruppe bestehend aus Haustiernahrungen, Ernährungsdiäten für Haustiere, Ergänzungsstoffe für Haustiere, Leckerli für Haustiere und Futterspielzeug für Haustiere, wie z.B. Beißspielzeug und verzehrbares Spielzeug, ausgewählt ist.

6. Verfahren gemäß einem der vorhergehenden Ansprüche, wobei die Haustierfutterzusammensetzung weiterhin Präbiotika enthält, beispielsweise Oligofructose und Inulin.

7. Verfahren gemäß einem der vorhergehenden Ansprüche, wobei in der Haustierfutterzusammensetzung mindestens 95%, vorzugsweise mindestens 98%, weiter bevorzugt mindestens 99%, höchst bevorzugt mindestens 99,9%, idealerweise die gesamten Probiotika nicht replizierend sind.

8. Verfahren gemäß einem der vorhergehenden Ansprüche, wobei die probiotischen Mikroorganismen aus der Gruppe bestehend aus Bifidobacteria, Lactobacilli, Propionibacteria oder Kombinationen aus diesen, beispielsweise Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus reuteri, Lactobacillus rhamnosus, Lactobacillus johnsonii, Lactobacillus plantarum, Lactobacillus fermentum, Lactococcus lactis, Streptococcus thermophilics, Lactococcus lactis, Lactococcus diacetylactis, Lactococcus cremoris, Lactobacillus bulgaricus, Lactobacillus helveticus, Lactobacillus delbrueckii, Escherichia coli und/oder Mischungen aus diesen ausgewählt sind.

9. Verfahren gemäß einem der vorhergehenden Ansprüche, wobei die probiotischen Mikroorganismen aus der Gruppe bestehend aus Bifidobacterium longum NCC 3001 (ATCC BAA-999), Bifidobacterium longum NCC 2705 (CNCM I-2618), Bifidobacterium breve NCC 2950 (CNCM I-3865), Bifidobacterium lactis NCC 2818 (CNCM I-3446), Lactobacillus johnsonii La 1 (CNCM I-1225), Lactobacillus paracasei NCC 2461 (CNCM I-2116), Lactobacillus rhamnosus NCC 4007 (CGMCC 1.3274), Lactobacillus reuteri DSM17983, Lactobacillus reuteri ATCC 55730, Streptococcus thermophilus NCC 2059 (CNCM I-4153), Lactobacillus casei NCC 1825 (ACA-DC 6002), Escherichia coli Nissle (DSM 6601), Lactobacillus bulgaricus NCC 15 (CNCM I-1198), Lactococcus lactis NCC 2287 (CNCM I-4154) oder Kombinationen aus diesen ausgewählt sind.

10. Verfahren gemäß einem der vorhergehenden Ansprüche, wobei die Haustierfutterzusammensetzung 0,005 mg bis 1000 mg von nicht replizierenden Mikroorganismen pro Tagesdosis enthält.

## Revendications

1. Procédé de préparation d'une composition alimentaire pour animaux de compagnie comprenant des micro-organismes probiotiques non réplicatifs en une quantité correspondant à 10⁶ à 10¹² ufc par portion, ledit procédé comprenant les étapes consistant à traiter des micro-organismes probiotiques avec un traitement à haute température et de courte durée (HTST) à une température de 120-140 °C pendant 1-30 secondes ou un traitement à ultra-haute température (UHT) à une température excédant 135 °C pendant 1-10 secondes, et ainsi rendre au moins 90% des micro-organismes non réplicatifs.

2. Procédé selon la revendication 1, dans lequel ladite composition alimentaire pour animaux de compagnie comprend de 4 à 40% en poids sec de matière grasse, de 12 à 70% en poids sec de glucides, et de 12 à 50% en poids sec de protéines.

3. Procédé selon la revendication 2, dans lequel ladite composition alimentaire pour animaux de compagnie comprend de 10 à 20% en poids sec de matière grasse, de 30 à 60% en poids sec de glucides, et de 20 à 35% en poids sec de protéines.

4. Procédé selon l'une quelconque des revendications précédentes, dans lequel ladite composition alimentaire pour animaux de compagnie comprend en outre de 0,5 à 40% en poids sec, de préférence de 0,5 à 30% en poids sec, plus préférablement de 1 à 20% en poids sec, le plus préférablement de 1 à 10% en poids sec de fibre alimentaire.

5. Procédé selon l'une des revendications précédentes, dans lequel ladite composition alimentaire pour animaux de compagnie est choisie parmi le groupe constitué d'aliments pour animaux de compagnie, de diètes nutritionnelles pour animaux de compagnie, de compléments alimentaires pour animaux de compagnie, de friandises pour animaux de compagnie, et de jouets de nourriture pour animaux de compagnie comme par exemple des jouets aptes à être mâcher et consommer.

6. Procédé selon l'une quelconque des revendications précédentes, dans lequel ladite composition alimentaire pour animaux de compagnie comprend en outre des prébiotiques, par exemple de l'oligofructose et de l'inuline.

7. Procédé selon l'une quelconque des revendications précédentes, dans lequel dans ladite composition alimentaire pour animaux de compagnie au moins 95%, de préférence au moins 98%, plus préférablement au moins 99%, le plus préférablement au moins 99,9%, idéalement tous les probiotiques sont non réplicatifs.

8. Procédé selon l'une quelconque des revendications précédentes, dans lequel les micro-organismes probiotiques sont choisis parmi le groupe constitué de bifidobactéries, lactobacilles, bactéries propioniques, ou combinaisons de ceux-ci, par exemple Bifidobacterium longum, Bifidobacterium lactis, Bifidobacterium animalis, Bifidobacterium breve, Bifidobacterium infantis, Bifidobacterium adolescentis, Lactobacillus acidophilus, Lactobacillus casei, Lactobacillus paracasei, Lactobacillus salivarius, Lactobacillus reuteri, Lactobacillus rhamnosus, Lactobacillus johnsonii, Lactobacillus plantarum, Lactobacillus fermentum, Lactococcus lactis, Streptococcus thermophilics, Lactococcus lactis, Lactococcus diacetylactis, Lactococcus cremoris, Lactobacillus bulgaricus, Lactobacillus helveticus, Lactobacillus delbrueckii, Escherichia coli et/ou mélanges de ceux-ci.

9. Procédé selon l'une quelconque des revendications précédentes, dans lequel les micro-organismes probiotiques sont choisis parmi le groupe constitué de Bifidobacterium longum NCC 3001 (ATCC BAA-999), Bifidobacterium longum NCC 2705 (CNCM I-2618), Bifidobacterium breve NCC 2950 (CNCM I-3865), Bifidobacterium lactis NCC 2818 (CNCM I-3446), Lactobacillus johnsonii La 1 (CNCM I-1225), Lactobacillus paracasei NCC 2461 (CNCM I-2116), Lactobacillus rhamnosus NCC 4007 (CGMCC 1.3274), Lactobacillus reuteri DSM17983, Lactobacillus reuteri ATCC 55730, Streptococcus thermophilus NCC 2059 (CNCM I-4153), Lactobacillus casei NCC 1825 (ACA-DC 6002), Escherichia coli Nissle (DSM 6601), Lactobacillus bulgaricus NCC 15 (CNCM I-1198), Lactococcus lactis NCC 2287 (CNCM I-4154), ou combinaisons de ceux-ci.

10. Procédé selon l'une quelconque des revendications précédentes, dans lequel ladite composition alimentaire pour animaux de compagnie contient 0005 mg - 1000 mg de micro-organismes non réplicatifs par dose quotidienne.
